(19) Europäisches Patentamt
European Patent Office
Office européen des brevets

(11) **EP 2 617 292 A1**

(12) **EUROPEAN PATENT APPLICATION**
published in accordance with Art. 153(4) EPC

(43) Date of publication:
**24.07.2013 Bulletin 2013/30**

(51) Int Cl.:
***A23D 9/007*** *(2006.01)*

(21) Application number: **11825147.9**

(22) Date of filing: **13.09.2011**

(86) International application number:
**PCT/JP2011/070798**

(87) International publication number:
**WO 2012/036145 (22.03.2012 Gazette 2012/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2010 JP 2010205276**

(71) Applicant: **Kao Corporation
Chuo-Ku
Tokyo 103-8210 (JP)**

(72) Inventors:
• **SAITO, Katsuyoshi**
**Tokyo 131-8501 (JP)**
• **MITSUI, Yuki**
**Tokyo 131-8501 (JP)**
• **MORIWAKI, Junya**
**Tokyo 131-8501 (JP)**

(74) Representative: **HOFFMANN EITLE
Patent- und Rechtsanwälte
Arabellastrasse 4
81925 München (DE)**

(54) **OIL AND FAT COMPOSITION**

(57)    Provided is adiacylglycerol-containing fat or oil composition in which a precipitation of crystal is inhibited even under low temperature. The fat or oil composition comprises the following components (A) and (B) : (A) a polyglycerin fatty acid ester having an average polymerization degree of glycerin of 20 or more; and (B) 20 mass% or more of diacylglycerol.

EP 2 617 292 A1

## Description

Field of the Invention

The present invention relates to a fat or oil composition containing diacylglycerol.

Background of the Invention

It has been found that diacylglycerol has an effect of improving a blood cholesterol level (see Patent Document 1) and has an effect of reducing accumulation of body fat, thereby preventing obesity (see Patent Document 2). This is probably because an intake of the diacylglycerol contributes to inhibiting an increase in a postprandial blood neutral fat level. On the other hand, the diacylglycerol is liable to crystallize under low temperature because of having a higher melting point than triacylglycerol.

A method using a polyglycerin fatty acid ester is known as a method of inhibiting crystallization of a fat or oil at low cost. There are disclosed, for example, a method using a polyglycerin fatty acid ester having an average polymerization degree of glycerin of from 2 to 15 and containing unsaturated fatty acids at 4 mass% or less in its constituent fatty acids (see Patent Document 3), a method using a polyglycerin fatty acid ester having an average polymerization degree of glycerin of from 2 to 15 and containing erucic acid as a main component in its constituent fatty acids (see Patent Document 4), and a method using an esterified product of polyglycerin and fatty acids, the esterified product having a hydroxyl value of 100 mg KOH/g or less and the polyglycerin having a hydroxy value of 850 mg KOH/g or less and having a content of a primary hydroxyl group of 50% or more (see Patent Document 5).

Citation List

Patent Document

[Patent Document 1] WO 99/48378 A1
[Patent Document 2] JP-A-4-300826
[Patent Document 3] JP-A-11-279115
[Patent Document 4] JP-A-2002-212587
[Patent Document 5] WO 2010/010953 A1

Summary of the Invention

The present invention relates to the following items (1) to (31). Regarding the embodiment as mentioned below, the present invention also includes other embodiments described below, related to the composition.

(1) A fat or oil composition, comprising the following components (A) and (B):

(A) a polyglycerin fatty acid ester having an average polymerization degree of glycerin of 20 or more; and
(B) 20 mass% or more of diacylglycerol.

(2) The fat or oil composition according to the above-mentioned item (1), in which the polyglycerin fatty acid ester has a hydroxyl value of 80 mg-KOH/g or less.
(3) The fat or oil composition according to the above-mentioned item (1), in which the polyglycerin fatty acid ester has a hydroxyl value of from 0 to 60 mg-KOH/g.
(4) The fat or oil composition according to the above-mentioned item (1), in which the polyglycerin fatty acid ester has a hydroxyl value of from 3 to 50 mg-KOH/g.
(5) The fat or oil composition according to the above-mentioned item (1), in which the polyglycerin fatty acid ester has a hydroxyl value of from 5 to 30 mg-KOH/g.
(6) The fat or oil composition according to the above-mentioned item (1), in which the polyglycerin fatty acid ester has a hydroxyl value of from 5 to 20 mg-KOH/g.
(7) The fat or oil composition according to any one of the above-mentioned items (1) to (6), in which constituent fatty acids of the polyglycerin fatty acid ester comprise the following (a-1), (a-2), and (a-3):

(a-1) from 20 to 80 mass% of saturated fatty acids having 16 or more carbon atoms;

(a-2) from 0 to 80 mass% of saturated fatty acids having 14 or less carbon atoms; and
(a-3) from 0 to 80 mass% of unsaturated fatty acids having 16 or more carbon atoms.

(8) The fat or oil composition according to any one of the above-mentioned items (1) to (6), in which constituent fatty acids of the polyglycerin fatty acid ester comprise the following (a-1), (a-2), and (a-3):

(a-1) from 20 to 80 mass% of saturated fatty acids having 16 or more carbon atoms;
(a-2) from 0 to 40 mass% of saturated fatty acids having 14 or less carbon atoms; and
(a-3) from 0 to 80 mass% of unsaturated fatty acids having 16 or more carbon atoms.

(9) The fat or oil composition according to any one of the above-mentioned items (1) to (6), in which constituent fatty acids of the polyglycerin fatty acid ester comprise the following (a-1), (a-2), and (a-3):

(a-1) from 20 to 80 mass% of saturated fatty acids having 16 or more carbon atoms;
(a-2) from 0 to 38 mass% of saturated fatty acids having 14 or less carbon atoms; and
(a-3) from 0 to 70 mass% of unsaturated fatty acids having 16 or more carbon atoms.

(10) The fat or oil composition according to any one of the above-mentioned items (1) to (6), in which constituent fatty acids of the polyglycerin fatty acid ester comprise the following (a-1), (a-2), and (a-3):

(a-1) from 20 to 70 mass% of saturated fatty acids having 16 or more carbon atoms;
(a-2) from 10 to 38 mass% of saturated fatty acids having 14 or less carbon atoms; and
(a-3) from 0 to 60 mass% of unsaturated fatty acids having 16 or more carbon atoms.

(11) The fat or oil composition according to any one of the above-mentioned items (1) to (6), in which constituent fatty acids of the polyglycerin fatty acid ester comprise the following (a-1), (a-2), and (a-3):

(a-1) from 20 to 60 mass% of saturated fatty acids having 16 or more carbon atoms;
(a-2) from 10 to 38 mass% of saturated fatty acids having 14 or less carbon atoms; and
(a-3) from 20 to 60 mass% of unsaturated fatty acids having 16 or more carbon atoms.

(12) The fat or oil composition according to any one of the above-mentioned items (1) to (6), in which constituent fatty acids of the polyglycerin fatty acid ester comprise the following (a-1), (a-2), and (a-3):

(a-1) from 30 to 45 mass% of saturated fatty acids having 16 or more carbon atoms;
(a-2) from 20 to 35 mass% of saturated fatty acids having 14 or less carbon atoms; and
(a-3) from 25 to 40 mass% of unsaturated fatty acids having 16 or more carbon atoms.

(13) The fat or oil composition according to any one of the above-mentioned items (1) to (12), in which constituent fatty acids of the diacylglycerol comprise from 30 to 90 mass% of oleic acid.
(14) The fat or oil composition according to any one of the above-mentioned items (1) to (12), in which constituent fatty acids of the diacylglycerol comprise from 35 to 85 mass% of oleic acid.
(15) The fat or oil composition according to any one of the above-mentioned items (1) to (12), in which constituent fatty acids of the diacylglycerol comprise from 40 to 80 mass% of oleic acid.
(16) The fat or oil composition according to any one of the above-mentioned items (1) to (12), in which constituent fatty acids of the diacylglycerol comprise from 45 to 75 mass% of oleic acid.
(17) The fat or oil 1 composition according to any one of the above-mentioned items (1) to (12), in which constituent fatty acids of the diacylglycerol comprise from 50 to 75 mass% of oleic acid.
(18) The fat or oil composition according to any one of the above-mentioned items (1) to (12), in which constituent fatty acids of the diacylglycerol comprise from 55 to 75 mass% of oleic acid.
(19) The fat or oil composition according to any one of the above-mentioned items (1) to (12), in which constituent fatty acids of the diacylglycerol comprise from 60 to 75 mass% of oleic acid.
(20) The fat or oil composition according to any one of the above-mentioned items (1) to (19), in which a content of the polyglycerin fatty acid ester in the fat or oil composition is from 0.001 to 5 mass%.
(21) The fat or oil composition according to any one of the above-mentioned items (1) to (19), in which a content of the polyglycerin fatty acid ester in the fat or oil composition is from 0.005 to 5 mass%.
(22) The fat or oil composition according to any one of the above-mentioned items (1) to (19), in which a content of the polyglycerin fatty acid ester in the fat or oil composition is from 0.01 to 1 mass%.

(23) The fat or oil composition according to any one of the above-mentioned items (1) to (19), in which a content of the polyglycerin fatty acid ester in the fat or oil composition is from 0.03 to 1 mass%.

(24) The fat or oil composition according to any one of the above-mentioned items (1) to (19), in which a content of the polyglycerin fatty acid ester in the fat or oil composition is from 0.05 to 0.5 mass%.

(25) The fat or oil composition according to any one of the above-mentioned items (1) to (19), in which a content of the polyglycerin fatty acid ester in the fat or oil composition is from 0.1 to 0.3 mass%.

(26) The fat or oil composition according to any one of the above-mentioned items (1) to (25), in which the polyglycerin fatty acid ester has an average polymerization degree of glycerin of from 20 to 45.

(27) The fat or oil composition according to any one of the above-mentioned items (1) to (25), in which the polyglycerin fatty acid ester has an average polymerization degree of glycerin of from 25 to 45.

(28) The fat or oil composition according to any one of the above-mentioned items (1) to (25), in which the polyglycerin fatty acid ester has an average polymerization degree of glycerin of from 31 to 45.

(29) The fat or oil composition according to any one of the above-mentioned items (1) to (28), in which the content of the diacylglycerol in the fat or oil composition is from 20 to 99.5 mass%.

(30) The fat or oil composition according to any one of the above-mentioned items (1) to (28), in which the content of the diacylglycerol in the fat or oil composition is from 25 to 98 mass%.

(31) The fat or oil composition according to any one of the above-mentioned items (1) to (28), in which the content of the diacylglycerol in the fat or oil composition is from 30 to 95 mass%. Embodiment for Carrying out the Invention

However, the inventor found that the known technologies for inhibiting crystallization do not provide a sufficient inhibitory effect on the crystallization in some cases.

Thus, the present invention provides a diacylglycerol-containing fat or oil composition whose crystallization is inhibited even under low temperature.

The inventors of the present invention have made intensive studies to solve the above-mentionedproblem. As a result, the inventors found that a polyglycerin fatty acid ester having an average polymerization degree of glycerin of 20 or more exerts an excellent crystallization-inhibiting effect on diacylglycerol, thus improving the low temperature resistance of the diacylglycerol-containing fat or oil composition.

According to the present invention, there can be provided a diacylglycerol-containing fat or oil composition whose crystallization is inhibited under low temperature irrespective of the content of diacylglycerol.

A polyglycerin fatty acid ester, which serves as a component (A) to be used for the fat or oil composition of the present invention, is obtained by esterifying polyglycerin and fatty acids. The polyglycerin fatty acid ester has an average polymerization degree of glycerin of 20 or more, preferably from 20 to 45, more preferably from 25 to 45, more preferably from 25 to 40, more preferably from 30 to 40, even more preferably 31 to 45, from the standpoint of inhibiting the crystallization of diacylglycerol (hereinafter, also referred to as "DAG") and favorably maintaining the outer appearance of the fat or oil composition containing the diacylglycerol. The term "average polymerization degree of glycerin" refers to a value obtained by measurement by GPC of the polymerization degree of the polyglycerin part of a polyglycerin fatty acid ester.

The constituent fatty acids of the polyglycerin fatty acid ester include (a-1) saturated fatty acids having 16 or more carbon atoms at preferably from 20 to 80 mass% (hereinafter, simply described as "%") of the total amount of the fatty acids, more preferably from 20 to 70%, more preferably from 20 to 60%, more preferably from 25 to 50%, even more preferably from 30 to 45%, from the standpoint of inhibiting the crystallization of diacylglycerol and favorably maintaining the outer appearance. The constituent fatty acids of the polyglycerin fatty acid ester include (a-2) saturated fatty acids having 14 or less carbon atoms at preferably from 0 to 80% of the total amount of the fatty acids, more preferably from 0 to 40%, more preferably from 0 to 38%, more preferably from 10 to 60%, more preferably from 10 to 38%, even more preferably from 20 to 35%, from the standpoint of inhibiting the crystallization of diacylglycerol and favorably maintaining the outer appearance. The constituent fatty acids of the polyglycerin fatty acid ester include (a-3) unsaturated fatty acids having 16 or more carbon atoms at preferably from 0 to 80% of the total amount of the fatty acids, more preferably from 0 to 70%, more preferably from 0 to 60%, more preferably from 20 to 60%, more preferably from 25 to 50%, even more preferably from 25 to 40%, from the standpoint of inhibiting the crystallization of diacylglycerol and favorably maintaining the outer appearance.

That is, the constituent fatty acids of the polyglycerin fatty acid ester preferably include (a-1) the saturated fatty acids having 16 or more carbon atoms at from 20 to 80% of the total amount of the fatty acids, (a-2) the saturated fatty acids having 14 or less carbon atoms at from 0 to 80% of the total amount of the fatty acids, and (a-3) the unsaturated fatty acids having 16 or more carbon atoms at from 0 to 80% of the total amount of the fatty acids, more preferably include the saturated fatty acids having 16 or more carbon atoms at from 20 to 80% of the total amount of the fatty acids, the saturated fatty acids having 14 or less carbon atoms at from 0 to 40% of the total amount of the fatty acids, and the unsaturated fatty acids having 16 or more carbon atoms at from 0 to 80% of the total amount of the fatty acids, more preferably include the saturated fatty acids having 16 or more carbon atoms at from 20 to 80% of the total amount of the fatty acids, the saturated fatty acids having 14 or less carbon atoms at from 0 to 38% of the total amount of the fatty acids, and the unsaturated fatty acids having 16 or more carbon atoms at from 0 to 70% of the total amount of the fatty acids, more preferably include the saturated fatty acids having 16 or more carbon atoms at from 20 to 70% of the total amount of the fatty acids, the saturated fatty acids having 14 or less carbon atoms at from 10 to 38% of the total amount of the fatty acids, and the unsaturated fatty acids having 16 or more carbon atoms at from 0 to 60% of the total amount of the fatty acids, more preferably include the saturated fatty acids having 16 or more carbon atoms at from 20 to 60% of the total amount of the fatty acids, the saturated fatty acids having 14 or less carbon atoms at from 10 to 38% of the total amount of the fatty acids, and the unsaturated fatty acids having 16 or more carbon atoms at from 20 to 60% of the total amount of the fatty acids, and even more desirably include the saturated fatty acids having 16 or more carbon atoms at from 30 to 45% of the total amount of the fatty acids, the saturated fatty acids having 14 or less carbon atoms at from 20 to 35% of the total amount of the fatty acids, and the unsaturated fatty acids having 16 or more carbon atoms at from 25 to 40% of the total amount of the fatty acids, from the standpoint of inhibiting the crystallization of diacylglycerol and favorably maintaining the outer appearance.

As the saturated fatty acids having 16 or more carbon atoms, saturated fatty acids having from 16 to 22 carbon atoms are preferred, and palmitic acid and stearic acid are more preferred. As the saturated fatty acids having 14 or less carbon atoms, saturated fatty acids having from 6 to 14 carbon atoms are preferred, and capric acid, lauric acid, and myristic acid are more preferred. As the unsaturated fatty acids having 16 or more carbon atoms, unsaturated fatty acids having from 16 to 22 carbon atoms are preferred, and examples thereof include oleic acid, linoleic acid, and erucic acid. Of those, oleic acid is preferred. The constituent fatty acids of the polyglycerin fatty acid ester may be a single kind of fatty acid or may be a mixture of two or more kinds of fatty acids.

The polyglycerin fatty acid ester in the present invention has a hydroxyl value of preferably 80 mg-KOH/g or less, more preferably from 0 to 60 mg-KOH/g, more preferably from 3 to 50 mg-KOH/g, more preferably from 5 to 30 mg-KOH/g, even more preferably from 5 to 20 mg-KOH/g, from the standpoint of inhibiting the crystallization of diacylglycerol and favorably maintaining the outer appearance. The hydroxyl value in the present invention refers to a value obtained by measurement in accordance with Standard Methods for the Analysis of Fats, Oils and Related Materials described in Examples.

The content of the polyglycerin fatty acid ester in the fat or oil composition is preferably 0.001% or more, more preferably 0.005% or more, more preferably 0.01% or more, more preferably 0.03% or more, more preferably 0.05% or more, even more preferably 0.1% or more, from the standpoint of inhibiting the crystallization of diacylglycerol. Further, the content of the polyglycerin fatty acid ester in the fat or oil composition is, from the standpoint of its taste and flavor, preferably 5% or less, more preferably 1% or less, more preferably 0.5% or less, even more preferably 0.3% or less. The content of the polyglycerin fatty acid ester in the fat or oil composition is preferably from 0.001 to 5%, more preferably from 0.005 to 5%, more preferably from 0.01 to 1%, more preferably from 0.03 to 1%, more preferably from 0.05 to 0.5%, even more preferably from 0.1 to 0.3%, from the standpoints of the inhibition of the crystallization of diacylglycerol, the taste and flavor, and cookability. Two or more of polyglycerin fatty acid esters may be used in combination.

The fat or oil composition of the present invention contains a fat or oil. The "fat or oil" in the present invention includes one or more of glycerols including triacylglycerol, diacylglycerol, and monoacylglycerol.

The content of the fat or oil in the present invention is preferably 85% or more in the fat or oil composition of the present invention, more preferably from 90 to 99.999%, more preferably from 95 to 99.5%, even more preferably from 97 to 99%, from the standpoints of physiological effects, industrial productivity, and the outer appearance.

The fat or oil composition of the present invention contains diacylglycerol as a component (B) at 20% or more, and contains diacylglycerol at preferably 25% or more, more preferably 30% or more, from the standpoint of the physiological effects. Further, the content of diacylglycerol as the component (B) in the fat or oil composition of the present invention is preferably 99.5% or less, more preferably 98% or less, even more preferably 95% or less, from the standpoint of improving the industrial productivity. The content of diacylglycerol as the component (B) in the fat or oil composition of the present invention is preferably from 20 to 99.5%, more preferably from 25 to 98%, even more preferably from 30 to 95%, from the standpoints of the outer appearance, the industrial productivity, and the physiological effects.

The fat or oil composition of the present invention contains triacylglycerol at preferably from 1 to 80%, more preferably from 5 to 80%, even more preferably from 10 to 80%, from the standpoints of the physiological effects, the industrial productivity, and the outer appearance. Further, the content of monoacylglycerol is preferably 2% or less, more preferably from 0.01 to 1.5%, and the content of a free fatty acid (salt) is preferably 3.5% or less, more preferably from 0.01 to 1.5%, from the standpoint of the taste and flavor or the like. The constituent fatty acids of the triacylglycerol and the monoacylglycerol are preferably the same constituent fatty acids as those of the diacylglycerol from the standpoints of improving the physiological effects and the industrial productivity of the fat or oil composition.

The content of an unsaturated fatty acid in the constituent fatty acids of the diacylglycerol is preferably from 80 to 100%, more preferably from 85 to 99%, even more preferably from 90 to 98%, from the standpoints of the outer appearance and the physiological effects. The unsaturated fatty acid has preferably from 14 to 24 carbon atoms, more preferably from 16 to 22 carbon atoms, from the standpoint of the physiological effects.

The content of oleic acid in the constituent fatty acids of the diacylglycerol is preferably from 30 to 90%, more preferably from 40 to 80%, more preferably from 45 to 75%, more preferably from 55 to 75%, even more preferably from 60 to 75%, from the standpoint of effectively exerting the effects of the present invention, thus improving the outer appearance and the taste and flavor, and the physiological effects.

The content of a trans-unsaturated fatty acid in the constituent fatty acids of the diacylglycerol is preferably from 0.01 to 5%, more preferably from 0.01 to 3.5%, even more preferably from 0.01 to 3%, from the standpoints of the physiological effects and the outer appearance. Further, the content of fatty acids having 12 or less carbon atoms is preferably 5% or less, more preferably from 0 to 2%, even more preferably from 0 to 1%, from the standpoint of the taste and flavor.

A fat or oil containing diacylglycerol maybe originated from any of a vegetable fat or oil and an animal fat or oil. As specific raw materials for the fat or oil, there may be given rapeseed oil (canola oil), sunflower oil, corn oil, soybean oil, linseed oil, rice oil, safflower oil, cottonseed oil, palm oil, coconut oil, olive oil, grapeseed oil, abocado oil, sesame oil, peanut oil, macadamia nut oil, hazelnut oil, walnut oil, lard, beef tallow, chicken oil, butter oil, fish oil, or the like. Further, it is also possible to use, as a starting material, a fat or oil prepared by fractionating and mixing these oils and fats, or a fat or oil prepared by adjusting a composition of the fatty acid of any of these oils and fats through a hydrogenation, a transesterification reaction, or the like. Further, a nonhydrogenated fat or oil is preferred from the standpoint of reducing the content of a trans-unsaturated fatty acid in all the constituent fatty acids of the resultant fat or oil.

The fat or oil containing diacylglycerol may be obtained by an esterification reaction between fatty acids derived from any of the above-mentioned oils and fats and glycerin, a transesterification reaction (glycerolysis) between a fat or oil and glycerin, or the like. These reactions may be carried out by a chemical reaction using an alkali catalyst or the like, and are preferably carried out under an enzymatically mild condition by using a 1,3-selective lipase or the like, from the standpoint of, for example, the taste and flavor.

The fat or oil composition of the present invention preferably contains an antioxidant. The content of the antioxidant in the fat or oil composition is preferably from 0.005 to 0.5%, more preferably from 0.04 to 0.25%, even more preferably from 0.08 to 0.2%, from the standpoints of, for example, its taste and flavor, oxidation stability, and suppression of coloration. Any antioxidant which is usually used in a food may be used as the antioxidant. It is possible to use, for example, vitamin E, butylhydroxytoluene (BHT), butylhydroxyanisole (BHA), t-butylhydroxyquinone (TBHQ), vitamin C or a derivative thereof, phospholipids, and natural antioxidants such as a rosemary extract.

The fat or oil composition of the present invention preferably contains a phytosterol. The content of the phytosterol in the fat or oil composition is preferably from 0.05 to 5%, more preferably from 0.3 to 4.7%, from the standpoints of a cholesterol-lowering effect and the outer appearance. In this case, examples of the phytosterol include free forms of $\alpha$-sitosterol, $\beta$-sitosterol, stigmasterol, campesterol, $\alpha$-sitostanol, $\beta$-sitostanol, stigmastanol, campestanol, cycloartenol, or the like, and ester forms such as fatty acid esters thereof.

The fat or oil composition of the present invention has the excellent property in term of outer appearance, workability, taste and flavor, or the like. Hence, the fat or oil composition can be used in the same way as a general edible fat or oil, and can be applied to various beverages and foods each produced by using a fat or oil.

Examples of the foods and beverages include a health food, a functional food and a food for specified health use or the like, for aiming at promotion of good health by exerting an anti-obesity function. Specific examples of the products include: bakery foods such as breads, cakes, cookies, pies, pizza crusts, and bakery mixes; oil-in-water type emulsions such as soup, sauce, dressing, mayonnaise, coffee cream (including powder form), ice cream, and whipped cream; water-in-oil type emulsions such as margarine, spread, and buttercream; snack foods such as potato chips; milk products such as chocolate, caramel, cheese, and yoghurt; and dough, enrober oils and fats, filling oils and fats, noodles, frozen foods, retort foods, beverages and roux or the like.

In the present invention, the polyglycerin fatty acid ester having an average polymerization degree of glycerin of 20 or more is added to the fat or oil containing diacylglycerol to exert an excellent crystallization-inhibiting effect on the diacylglycerol, thus improving the low temperature resistance of the fat or oil composition containing diacylglycerol at a high content.

When the content of oleic acid in the constituent fatty acids of diacylglycerol is high, it is liable to crystallize particularly under low temperature, but the present invention exerts an excellent crystallization-inhibiting effect even on a fat or oil having a high content of oleic acid.

Examples

(Analysis method)

(i) Glyceride composition of fat or oil

About 10 mg of a fat or oil sample and 0.5 mL of a trimethylsilylating agent ("Silylating Agent TH" manufactured by Kanto Chemical Co., Inc.) were loaded into a glass sample bottle, followed by hermetical sealing, and the glass sample bottle was heated at 70°C for 15 minutes. 1.0 mL of water and 1.5 mL of hexane were added to the mixture, followed by shaking. It was left to stand still, and then its upper layer was subjected to gas-liquid chromatography (GLC) to perform analysis.

(ii) Composition of costituent fatty acids of fat or oil

In accordance with "Preparation of methyl esters of fatty acids (2.4.1. -1996) " in "The JOCS Standard Methods for the Analysis of Fats, Oils and Related Materials" edited by Japan Oil Chemists' Society, methyl esters of fatty acids were prepared. The resultant samples were measured by American Oil Chemists' Society Official Method Ce If-96 (GLC method).

(iii) Hydroxyl value of polyglycerin fatty acid ester

In accordance with "hydroxyl value (pyridine-acetic anhydride method 2.3.6.2-1996) " in "Standard Methods for the Analysis of Fats, Oils and Related Materials, 2003" edited by Japan Oil Chemists' Society, the hydroxyl value of each polyglycerin fatty acid ester was calculated. About 5 g of a fat or oil sample were weighed in a round-bottom flask with a long neck. 5 ml of an acetylating reagent were added to the fat or oil sample, and a small funnel was put in the neck of the flask. The bottomportion of the flask was immersed in a heating bath up to a depth of about 1 cm and was heated to a temperature of from 95 to 100°C. One hour later, the flask was taken out from the heating bath and was cooled. 1 ml of distilled water was added into the flask through the funnel, and the flask was heated again in the heating bath for 10 minutes. The flask was cooled again to normal temperature, and the liquid condensed on the inside surface of the funnel and on the inside surface of the neck of the flask was washed down into the flask with 5 ml of neutral ethanol. The resultant solution was subjected to titration with a 0.5 mol/L potassium hydroxide-ethanol reference solution by using a phenolphthalein indicator. Note that a blank test was performed simultaneously with the main test, and a value calculated from the results of the titration on the basis of the following equation was defined as "hydroxyl value (mg-KOH/g)" (OHV).

$$\text{Hydroxyl value} = (A-B) \times 28.05 \times F1/C + \text{acid value}$$

(A: Amount (ml) of a 0.5 mol/L potassium hydroxide-ethanol reference solution used in a blank test, B: Amount (ml) of a 0.5 mol/L potassium hydroxide-ethanol reference solution used in a main test, F1 : Factor of a 0.5 mol/L potassium hydroxide-ethanol reference solution, and C: Collection amount (g) of a sample)

The acid value of each polyglycerin fatty acid ester was calculated in accordance with "Acid value (2.3.1-1996) " in "Standard Methods for the Analysis of Fats, Oils and Related Materials, 2003" edited by Japan Oil Chemists' Society. About 5 g of a sample were weighed in a conical flask, and 100 mL of a solvent of ethanol: ethyl acetate=1.1 where added to the sample, followed by the dissolution of the sample. The solution was subjected to titration with a 0.1 mol/L potassium hydroxide-ethanol reference solution by using a phenolphthalein indicator, and a value calculated from the results of the titration on the basis of the following equation was defined as "acid value (mg-KOH/g)."

$$\text{Acid value} = 5.611 \times D \times F2/E$$

(D: Use amount (ml) of a 0.1 mol/L potassium hydroxide-ethanol reference solution, E: Collection amount (g) of a sample, and F2: Factor of a 0.1 mol/L potassium hydroxide-ethanol reference solution)

(iv) Isolation of constituent components of polyglycerin fatty acid ester

A polyglycerin fatty acid ester was separated into a polyglycerin part and a fatty acid part in accordance with a method described in "polyglycerin ester (p. 75) " issued by Sakamoto Yakuhin Kogyo Co., Ltd. The resultant polyglycerin part was used for the analysis of the average polymerization degree of glycerin and the resultant fatty acidpart was used for the analysis of the constituent fatty acids of the polyglycerin fatty acid ester.

(v) Measurement method for average polymerization degree of glycerin in polyglycerin fatty acid ester

The polyglycerin was analyzed by GPC under the conditions of using TSK 2500 PWXL (TOSOH CORPORATION) as a column, using distilled water (in which trifluoroacetic acid was added at 0.1%) as a solvent, setting the flow rate to 1 mL/min, using RID as a detector, setting the temperature to 40°C, and setting the injection volume to 50 µL. A standard curve was prepared by using polyethylene glycol, to thereby measure the weight-average molecular weight (Mw2) of the polyglycerin in terms of polyethylene glycol and the molecular weight (Mw1) of glycerin. Subsequently, the conversion factor (F) of glycerin was calculated on the basis of the following equation (1).

$$F = 92 / Mw1 \quad (1)$$

(where F represents the conversion factor of glycerin and Mw1 represents the molecular weight of glycerin.)
The "average polymerization degree of glycerin" in the polyglycerin was calculated by using the weight-average molecular weight (Mw2) calculated as described above in the following equation (2).

$$n = (Mw2 \times F - 18) / 74 \quad (2)$$

(where n represents the weight-average polymerization degree of glycerin, F represents the conversion factor of glycerin, and Mw2 represents the weight-average molecular weight of polyglycerin.)

(vi) Composition of constituent fatty acids of polyglycerin fatty acid ester

The composition of the constituent fatty acids of each polyglycerin fatty acid ester was measured by the same method as the composition of the constituent fatty acids of each fat or oil.

(Polyglycerin fatty acid ester)

From PGE 1 to PGE 9 (manufactured by Taiyo Kagaku Co., Ltd.) and the decaglycerin fatty acid ester THL-15 (manufactured by Sakamoto Yakuhin Kogyo Co., Ltd.) were each used as a polyglycerin fatty acid ester. Further, PGE 4 and PGE 9 were mixed, yielding PGE 10 and PGE 11. Table 1 shows the average polymerization degree of glycerin in each polyglycerin fatty acid ester, the hydroxyl value thereof, and the composition of fatty acid thereof.
[Table 1]

| PGE No. | Polymerization degree of glycerin in polyglycerin | Hydroxyl value [mg-KOH/g] | Composition of fatty acid (%) | | | | | |
|---|---|---|---|---|---|---|---|---|
| | | | C10:0 | C12:0 | C14:0 | C16:0 | C18:0 | C18.1 |
| PGE 1 | 6 | 23.3 | | | | 50 | | 50 |
| PGE 2 | 10 | 17.6 | | | | 50 | | 50 |
| PGE 3 | 25 | 21.4 | | | | 50 | | 50 |
| PGE 4 | 36 | 24.2 | | | | 50 | | 50 |
| PGE 5 | 36 | 55.8 | | | | 50 | | 50 |
| PGE 6 | 25 | 18.4 | | 20 | 8 | 29 | 9 | 34 |
| PGE 7 | 36 | 18.4 | | 20 | 8 | 29 | 9 | 34 |
| PGE 8 | 36 | 17.4 | | 60 | | 20 | 20 | |
| PGE 9 | 36 | 14.0 | | 100 | | | | |
| PGE 10 | 36 | 18.1 | | 60 | | 20 | | 20 |
| PGE 11 | 36 | 20.1 | | 40 | | 30 | | 30 |
| THL 15 | 10 | 6.3 | | 20 | | 60 | | 20 |

(Preparation of fat or oil containing DAG at high content)

Rapeseed oil was hydrolyzed, yielding fatty acids. 564 g of the fatty acids and 92 g of glycerin were mixed, and the mixture was subjected to an esterification reaction by using an immobilized 1,3-selective lipase (manufactured by Novo Nordisk Pharmaceutical Industries, Inc.) as a catalyst. After the lipase preparation was filtered off, the product after completion of the reaction was subjected to molecular distillation, followed by decoloration, water washing, and deodorization at 235°C for 1 hour, thereby yielding a fat or oil X.
Rapeseed oil was added to the fat or oil X to adjust the content of DAG, thereby yielding oils and fats Y and Z.

Soybean oil was hydrolyzed, yielding fatty acids, followed by wintering, thereby reducing the amount of saturated fatty acids therein. 395 g of the resultant fatty acids, 169 g of fatty acids obtained by hydrolyzing rapeseed oil, and 92 g of glycerin were mixed, and the mixture was subjected to an esterification reaction by using an immobilized 1,3-selective lipase (manufactured by Novo Nordisk Pharmaceutical Industries, Inc.) as a catalyst. After the lipase preparation was filtered off, the product after completion of the reaction was subjected to molecular distillation, followed by decoloration, water washing, and deodorization at 215°C for 1 hour, thereby yielding a fat or oil W.

Rapeseed oil was hydrolyzed, yielding fatty acids. 2,500 g of the fatty acids and 410 g of glycerin were mixed, and the mixture was subjected to an esterification reaction by using an immobilized 1,3-selective lipase (manufactured by Novo Nordisk Pharmaceutical Industries, Inc.) as a catalyst. After the lipase preparation was filtered off, the product after completion of the reaction was subjected to molecular distillation, followed by decoloration, water washing, and deodorization at 235°C for 1 hour, thereby yielding a fat or oil V.

Table 2 shows the compositions of glyceride and the compositions of fatty acid in the resultant oils and fats X, Y, Z, W, and V.

[Table 2]

| Fat or oil | Composition of glyceride (%) | | | | Composition of fatty acid (%) | | | | | | |
|---|---|---|---|---|---|---|---|---|---|---|---|
| | DAG | TAG | MAG | FFA | C16:0 | C18:0 | C18:1 | C18:2 | C18:3 | C20:0 | Others |
| Fat or oil X | 71.6 | 27.5 | 0.8 | 0.1 | 3.9 | 2.0 | 61.4 | 19.2 | 11.0 | 0.8 | 1.7 |
| Fat or oil Y | 33.0 | 66.6 | 0.4 | 0.1 | 4.2 | 1.9 | 62.8 | 19.2 | 10.6 | 0.6 | 0.7 |
| Fat or oil Z | 22.5 | 77.2 | 0.2 | 0.1 | 4.1 | 1.9 | 63.1 | 19.2 | 10.4 | 0.6 | 0.7 |
| Fat or oil W | 86.3 | 12.8 | 0.7 | 0.2 | 2.7 | 1.0 | 37.3 | 48.9 | 8.9 | 0.3 | 0.9 |
| Fat or oil V | 84.1 | 14.6 | 1.2 | 0.1 | 4.1 | 1.9 | 60.3 | 19.8 | 11.6 | 0.6 | 1.7 |

TAG: Triacylglycerol
DAG: Diacylglycerol
MAG: Monoacylglycerol
FFA: Free fatty acid

(Preparation of fat or oil composition)

Any of from PGE 1 to PGE 7 and the decaglycerin fatty acid ester "THL-15" was added to the DAG-containing fat or oil X, Y, Z, or W, so as to account for 0.1% of the resultant fat or oil composition, thereby preparing DAG-containing fat or oil compositions of Examples 1 to 14 and Comparative Examples 1 to 16.
PGE 1, PGE 4, PGE 7, PGE 8, PGE 10, or PGE 11 was added to the DAG-containing fat or oil V so as to achieve the various concentrations shown in Table 7, thereby preparing DAG-containing fat or oil compositions of Examples 15 to 22 and Comparative Examples 17 and 18.

Each of these fat or oil compositions was poured in a 30-gram aliquot into a glass vial (SV - 50 manufactured by Nichiden-Rika Glass Co., Ltd.). The glass vial was closed with a lid, and left to stand still for 1 to 7 days in a refrigerator at 5°C (in the cases of Examples 1 to 5 and Comparative Examples 1 to 4 each prepared by using the fat or oil X), a refrigerator at 0°C (in the cases of Examples 6 to 14 and Comparative Examples 5 to 16 each prepared by using the fat or oil Y, Z, or W), or a refrigerator at 8°C (in the cases of Examples 15 to 22 and Comparative Examples 17 and 18 each prepared by using the fat or oil V). The presence or absence of crystals was checkedvisually every day on the basis of the criteria shown below. The results are shown in Tables 3 to 7.

(Evaluation criteria of presence or absence of crystals)

4: Clear
3: Slight crystal precipitation/haze
2: Crystal precipitation
1: Solidification

[Table 3]

| | | Comparative Example 1 | Comparative Example 2 | Comparative Example 3 | Comparative Example 4 | Example 1 | Example 2 | Example 3 | Example 4 | Example 5 |
|---|---|---|---|---|---|---|---|---|---|---|
| Fat or oil | | Fat or oil X | Fat or oil X | Fat or oil X | Fat or oil X | Fat or oil X | Fat or oil X | Fat or oil X | Fat or oil X | Fat or oil X |
| Added PGE | | Free of PGE | PGE 1 | PGE 2 | THL-15 | PGE 3 | PGE 4 | PGE 5 | PGE 6 | PGE 7 |
| Number of observation days (5 °C) | First day | 1 | 3 | 4 | 4 | 4 | 4 | 4 | 4 | 4 |
| | Second day | 1 | 3 | 3 | 3 | 4 | 4 | 4 | 4 | 4 |
| | Third day | 1 | 2 | 3 | 2 | 4 | 4 | 4 | 4 | 4 |
| | Fourth day | 1 | 2 | 2 | 2 | 4 | 4 | 4 | 4 | 4 |
| | Fifth day | 1 | 2 | 2 | 2 | 4 | 4 | 4 | 4 | 4 |
| | Sixth day | 1 | 2 | 2 | 2 | 4 | 4 | 3 | 4 | 4 |
| | Seventh day | 1 | 2 | 2 | 2 | 3 | 4 | 3 | 4 | 4 |

[Table 4]

| | | Comparative Example 5 | Comparative Example 6 | Comparative Example 7 | Comparative Example 8 | Example 6 | Example 7 | Example 8 |
|---|---|---|---|---|---|---|---|---|
| Fat or oil | | Fat or oil Y | Fat or oil Y | Fat or oil Y | Fat or oil Y | Fat or oil Y | Fat or oil Y | Fat or oil Y |
| Added PGE | | Free of PGE | PGE 1 | PGE 2 | THL-15 | PGE 3 | PGE 4 | PGE 5 |
| Number of observation days (0°C) | First day | 2 | 3 | 3 | 3 | 4 | 4 | 4 |
| | Second day | 2 | 3 | 3 | 3 | 4 | 4 | 4 |
| | Third day | 2 | 3 | 3 | 3 | 4 | 4 | 4 |
| | Fourth day | 2 | 2 | 3 | 2 | 4 | 4 | 4 |
| | Fifth day | 2 | 2 | 2 | 2 | 4 | 4 | 4 |

[Table 5]

| | | Comparative Example 9 | Comparative Example 10 | Comparative Example 11 | Comparative Example 12 | Example 9 | Example 10 | Example 11 |
|---|---|---|---|---|---|---|---|---|
| Fat or oil | | Fat or oil Z | Fat or oil Z | Fat or oil Z | Fat or oil Z | Fat or oil Z | Fat or oil Z | Fat or oil Z |
| Added PGE | | Free of PGE | PGE 1 | PGE 2 | THL-15 | PGE 3 | PGE 4 | PGE 5 |
| Number of observation days (0 °C) | First day | 3 | 4 | 4 | 4 | 4 | 4 | 4 |
| | Second day | 3 | 3 | 3 | 3 | 4 | 4 | 4 |
| | Third day | 3 | 3 | 3 | 3 | 4 | 4 | 4 |
| | Fourth day | 3 | 3 | 3 | 3 | 4 | 4 | 4 |
| | Fifth day | 3 | 3 | 3 | 3 | 4 | 4 | 4 |

EP 2 617 292 A1

[Table 6]

| | | Comparative Example 13 | Comparative Example 14 | Comparative Example 15 | Comparative Example 16 | Example 12 | Example 13 | Example 14 |
|---|---|---|---|---|---|---|---|---|
| Fat or oil | | Fat or oil W | Fat or oil W | Fat or oil W | Fat or oil W | Fat or oil W | Fat or oil W | Fat or oil W |
| Added PGE | | Free of PGE | PGE 1 | PGE 2 | THL-15 | PGE 3 | PGE 4 | PGE 5 |
| Number of observation days (0°C) | First day | 2 | 4 | 4 | 4 | 4 | 4 | 4 |
| | Second day | 1 | 3 | 4 | 3 | 4 | 4 | 4 |
| | Third day | 1 | 3 | 3 | 3 | 4 | 4 | 4 |
| | Fourth day | 1 | 3 | 3 | 3 | 4 | 4 | 4 |
| | Fifth day | 1 | 2 | 3 | 2 | 4 | 4 | 4 |

[Table 7]

| | | Comparative Example 17 | Comparative Example 18 | Example 15 | Example 16 | Example 17 | Example 18 | Example 19 | Example 20 | Example 21 | Example 22 |
|---|---|---|---|---|---|---|---|---|---|---|---|
| Fat or oil | | Fat or oil V | Fat or oil V | Fat or oil V | Fat or oil V | Fat or oil V | Fat or oil V | Fat or oil V | Fat or oil V | Fat or oil V | Fat or oil V |
| Added PGE | | Free of PGE | PGE 1 | PGE 4 | PGE 4 | PGE 4 | PGE 7 | PGE 7 | PGE 8 | PGE 10 | PGE 11 |
| Addition amount | | - | 0.1% | 0.02% | 0.1% | 0.3% | 0.02% | 0.1% | 0.1% | 0.1% | 0.1% |
| Number of observation days (8°C) | First day | 2 | 3 | 4 | 4 | 4 | 4 | 4 | 4 | 4 | 4 |
| | Second day | 2 | 3 | 3 | 4 | 4 | 4 | 4 | 4 | 4 | 4 |
| | Third day | 2 | 2 | 3 | 4 | 4 | 3 | 4 | 3 | 4 | 4 |
| | Fourth day | 2 | 2 | 3 | 4 | 4 | 3 | 4 | 3 | 3 | 4 |
| | Fifth day | 2 | 2 | 3 | 4 | 4 | 3 | 4 | 3 | 3 | 4 |
| | Sixth day | 2 | 2 | 3 | 4 | 4 | 3 | 4 | 3 | 3 | 4 |
| | Seventh day | 2 | 2 | 3 | 4 | 4 | 3 | 4 | 3 | 3 | 3 |

The results shown in Tables 3 to 7 confirmed that haze or crystallization occurred under low temperature within a short period of time in each of the fat or oil compositions of the comparative examples, and hence the fat or oil compositions didnot have sufficient low temperature resistance. On the other hand, the fat or oil compositions of the examples in each of which a polyglycerin fatty acid ester having an average polymerization degree of glycerin of 20 or more was used retained a clear state for a long period of time. Further, it demonstrated that, even when a DAG-containing fat or oil having a high content of oleic acid in its constituent fatty acids was used, the low temperature resistance of the resultant fat or oil composition improved. Particularly when any of PGE 4, 6, and 7 was used, the resultant fat or oil composition retained a clear state even after 7 days passed, and hence had remarkably improved low temperature resistance.

**Claims**

1. A fat or oil composition, comprising the following components (A) and (B) :

   (A) a polyglycerin fatty acid ester having an average polymerization degree of glycerin of 20 or more; and
   (B) 20 mass% or more of diacylglycerol.

2. The fat or oil composition according to claim 1, wherein the polyglycerin fatty acid ester has a hydroxyl value of 80 mg-KOH/g or less.

3. The fat or oil composition according to claim 1 or 2, wherein constituent fatty acids of the polyglycerin fatty acid ester comprise the following (a-1), (a-2), and (a-3):

   (a-1) from 20 to 80 mass% of saturated fatty acids having 16 or more carbon atoms;
   (a-2) from 0 to 80 mass% of saturated fatty acids having 14 or less carbon atoms; and
   (a-3) from 0 to 80 mass% of unsaturated fatty acids having 16 or more carbon atoms.

4. The fat or oil composition according to any one of claims 1 to 3 , wherein constituent fatty acids of the diacylglycerol comprise from 30 to 90 mass% of oleic acid.

5. The fat or oil composition according to any one of claims 1 to 4, wherein a content of the polyglycerin fatty acid ester in the fat or oil composition is from 0.001 to 5 mass%.

6. The fat or oil composition according to any one of claims 1 to 5, wherein the polyglycerin fatty acid ester has an average polymerization degree of glycerin of from 20 to 45.

7. The fat or oil composition according to any one of claims 1 to 6, wherein the content of the diacylglycerol in the fat or oil composition is from 20 to 99.5 mass%.

**INTERNATIONAL SEARCH REPORT**

| International application No. |
| --- |
| PCT/JP2011/070798 |

A. CLASSIFICATION OF SUBJECT MATTER
*A23D9/007*(2006.01)i

According to International Patent Classification (IPC) or to both national classification and IPC

B. FIELDS SEARCHED

Minimum documentation searched (classification system followed by classification symbols)
A23D9/007

Documentation searched other than minimum documentation to the extent that such documents are included in the fields searched
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X/Y | JP 2009-159829 A (Kao Corp.), 23 July 2009 (23.07.2009), claims; paragraph [0025]; tables 2, 6 (Family: none) | 1-7/1-7 |
| Y | US 2002/0119239 A1 (KAO CORP.), 29 August 2002 (29.08.2002), claims & EP 1214886 A1 & JP 2002-176952 A | 1-7 |
| Y | KURIYAMA J. et al., Effect of Polyglycerol Fatty Acid Esters on Resistance to Crystallization of Palm Olein, J. Oleo. Sci., Vol.50, No.10, 2001.10.01, pp.831-838 | 1-7 |

☒ Further documents are listed in the continuation of Box C.　　☐ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 01 December, 2011 (01.12.11) | Date of mailing of the international search report 13 December, 2011 (13.12.11) |
| Name and mailing address of the ISA/ Japanese Patent Office | Authorized officer |
| Facsimile No. | Telephone No. |

Form PCT/ISA/210 (second sheet) (July 2009)

**EP 2 617 292 A1**

<table>
<tr><td align="center"><strong>INTERNATIONAL SEARCH REPORT</strong></td><td>International application No.<br><br>PCT/JP2011/070798</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2010/010953 A1  (TAIYO KAGAKU CO., LTD.), 28 January 2010 (28.01.2010), abstract<br>& JP 4443628 B          & CA 2731569 A<br>& CN 102105576 A | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9948378 A1
- JP 4300826 A
- JP 11279115 A
- JP 2002212587 A
- WO 2010010953 A1

**Non-patent literature cited in the description**

- Preparation of methyl esters of fatty acids (2.4.1. -1996. The JOCS Standard Methods for the Analysis of Fats, Oils and Related Materials. 1996
- hydroxyl value (pyridine-acetic anhydride method 2.3.6.2-1996. Standard Methods for the Analysis of Fats, Oils and Related Materials. 2003
- Acid value (2.3.1-1996. Standard Methods for the Analysis of Fats, Oils and Related Materials. 2003